# FASCICULE DE BREVET EUROPEEN

(11) **EP 0 886 502 B1**
(45) Date de publication et mention de la délivrance du brevet: **15.12.1999**
(21) Numéro de dépôt: 97904978.0
(22) Date de dépôt: 11.03.1997
(51) Int. Cl.: A61F 2/08

(54) **DISPOSITIF PASSE-TENDON**
SEHNENDURCHFÜHRVORRICHTUNG
TENDON GUIDE DEVICE

(30) Priorité: 11.03.1996 FR 9603190
(43) Date de publication de la demande: 30.12.1998
(73) Titulaire: Rippstein, Pascal François, 8702 Zollikon (CH)
(72) Inventeur: Rippstein, Pascal François, 8702 Zollikon (CH)
(74) Mandataire: Nithardt, Roland
(86) Numéro de dépôt international: CH9700099
(87) Numéro de publication internationale: WO9733535

(56) Documents cités:
- EP-A- 0 145 492
- EP-A- 0 642 773
- WO-A-93/09730
- WO-A-96/03084
- FR-A- 2 678 823
- US-A- 5 451 203

## Description

La présente invention concerne un dispositif passe-tendon, pour passer un tendon dans un canal ossaire pouvant avoir une forme coudée, comprenant un élément de maintien d'une extrémité du tendon, ledit élément de maintien étant pourvu d'une tige souple pour faire passer ledit tendon dans ledit canal ossaire.

Dans le cas d'une rupture ligamentaire, par exemple au niveau du pied, de la main ou de toute autre articulation, la solution chirurgicale pour éviter une instabilité invalidante consiste soit à restaurer le tendon endommagé au moyen d'éléments chirurgicaux tels que par exemple ceux décrits par les publications WO-A-96 03084 et EP-A-0 145 492, soit à remplacer le ligament rompu par un tendon accessoire de peu d'importance fonctionnelle et qui pourra donc être prélevé sans causer de préjudice au patient ou par un tendon biologique de substitution. Un tel tendon, qui reproduit la fonction d'un ligament naturel, est par exemple décrit dans la publication EP-A- 0 642 773. Ce faux ligament se compose d'un coeur entouré par un fourreau flexible tubulaire réalisé en un treillis à mailles déformables de matière synthétique ou de matière biodégradable. On peut également procéder au renforcement du tendon endommagé au moyen d'un dispositif tel que celui objet de la publication FR-A-2 678 823 qui se présente sous la forme d'un manchon tricoté dans un fil résorbable destiné à envelopper le tendon endommagé et à générer une stimulation périphérique et un massage de celui-ci lui permettant de recréer des fibres.

Pour effectuer une restauration ou un remplacement, le tendon est passé dans des canaux ossaires, forés en général aussi près que possible des insertions originales du ligament rompu par exemple à l'aide d'un élément de traction tel que celui objet du brevet US 5 451 203 formé d'un tube fermé à une de ses extrémités et extensible ou rétractable sous l'action d'un lacet reliant ses deux extrémités. Le tendon forme ensuite une boucle simple, double, en "huit" ou ayant d'autres formes et remplace le ligament déchiré. D'autre part, dans la plupart des transferts musculo-tendineux qui sont réalisés pour différentes pathologies de la main et du pied, le ou les tendons transférés sont désinsérés de leur insertion distale et réinsérés sur un nouveau site, au travers d'un canal ossaire. Le tendon est passé au travers de ce canal, forme en général une boucle et sera suturé sur lui-même ou directement sur le périoste.

La difficulté majeure de ces interventions consiste bien souvent à passer le tendon dans les canaux ossaires d'un diamètre à peine supérieur au diamètre du tendon, la faible masse osseuse locale ne permettant pas de percer des canaux d'un diamètre beaucoup plus important. De plus, pour permettre une intégration rapide et solide du tendon dans l'os, il est souhaitable qu'il y ait un contact aussi intime que possible entre l'os et le tendon.

La méthode connue, classiquement utilisée, consiste tout d'abord à solidariser un fil serti sur une aiguille à l'extrémité du tendon. Pour garantir un ancrage aussi solide que possible, le fil est passé plusieurs fois dans le tendon. Ensuite, l'aiguille et le fil qui lui est attaché sont passés dans le canal ossaire et, enfin, le fil est tiré pour faire passer le tendon dans le canal ossaire.

Cette méthode présente de nombreux inconvénients. Tout d'abord, le fil devant être passé plusieurs fois dans le tendon, celui-ci peut être endommagé et affaibli. Cette atteinte est d'autant plus gênante que le tendon est court et qu'il doit pouvoir être utilisé au maximum de sa longueur. De plus, la structure d'un tendon correspond approximativement à celle d'une corde qui serait constituée de plusieurs fils, tous d'un diamètre à peu près identique. Du fait de cette structure, l'ancrage du fil n'est jamais très solide et il arrive fréquemment qu'il lâche lors de l'application de la force de traction nécessaire pour passer le tendon dans le canal ossaire. Cette rupture de l'ancrage augmente les dommages au tendon et rend un nouvel ancrage très précaire.

D'autre part, le canal ossaire est généralement foré de façon approximative au moyen d'une simple perceuse. Bien souvent, ce sont deux trous percés en V qui forment ces canaux et qui présentent donc des configurations très variables.

Par contre, les aiguilles présentent une configuration bien établie, légèrement modifiable par pliage. Si la configuration du canal ossaire et celle de l'aiguille ne correspondent pas, ce qui est souvent le cas, l'aiguille passera mal dans le canal. Le passage de l'aiguille est d'autant plus difficile que sa pointe a tendance à s'accrocher dans l'os spongieux qui constitue les parois du canal ossaire.

A ce stade, soit le chirurgien essaie de donner à l'aiguille une configuration identique à celle du canal et l'aiguille finit souvent par casser, soit il essaie de donner au canal ossaire une configuration identique à celle de l'aiguille et il finit souvent par provoquer une fracture qui rend le canal ossaire inutilisable.

Ensuite, une fois l'aiguille et le fil passés, il faut passer à son tour le tendon dans le canal ossaire. Cette étape représente souvent la difficulté majeure de l'intervention. En effet, l'extrémité du tendon qui arrive à l'entrée du canal ne passe souvent pas en entier dans le canal. En tirant sur le fil, on provoque un "bourgeonnement" du tendon qui augmente son diamètre. Le passage devient alors définitivement impossible. En tirant plus fort, on provoque finalement la rupture de l'ancrage. Dans tous ces cas, le tendon est endommagé et un nouvel ancrage n'est souvent possible qu'au prix fort de l'amputation de la partie lésée du tendon.

A ce stade, le chirurgien utilise souvent toutes sortes de dispositifs plus ou moins pointus pour essayer de faire pénétrer l'intégralité de l'extrémité du tendon dans l'orifice d'entrée du canal et exerce une traction de plus en plus forte. C'est d'ailleurs souvent à ce moment que l'ancrage lâche. Parfois, il essaie d'agrandir le trou au risque de provoquer une fracture et de rendre le canal ossaire inutilisable ou il amincit le tendon, ce qui lui fait perdre de sa résistance.

Cette méthode transforme une intervention initialement simple en un travail long et compliqué à cause de l'utilisation de dispositifs inadaptés.

Pour tenter de résoudre ces problèmes, on a utilisé un dispositif chirurgical constitué d'une petite gaine en forme de cône, faite en matière plastique souple, dans laquelle le tendon est glissé, puis ancré au moyen de fils qui le traversent de part en part. Le passage dans le canal ossaire est ainsi théoriquement facilité.

Cependant, cette technique présente plusieurs inconvénients. Plus particulièrement, la gaine occupera un volume non négligeable, et le fait d'augmenter le diamètre de l'ensemble tendon-gaine, peut rendre impossible son passage dans un canal ossaire. De plus, après le retrait de la gaine on n'obtient pas un contact os-tendon optimal car pas très intime.

D'autre part, le tendon doit être ancré à la gaine par du fil. Cet ancrage endommage le tendon et sa réalisation nécessite du temps. De plus, à la sortie du tendon, l'ancrage devra être défait alors qu'en cas d'ancrage direct sur le tendon, cette démarche supplémentaire est supprimée.

Enfin, cette technique nécessite l'utilisation de plusieurs formats de gaine selon les diverses tailles de tendon. De plus, les gaines ont un prix d'achat relativement élevé. Elles sont piquées et stérilisées plusieurs fois, s'usent et doivent donc être remplacées régulièrement. Le coût de cette technique est donc relativement élevé.

La présente invention se propose de remédier à ces inconvénients et a pour but de fournir un dispositif passe-tendon d'utilisation simple et rapide, qui ne porte pas atteinte à l'intégrité du tendon, qui est d'un coût particulièrement avantageux et, surtout, qui rend l'intervention consistant à mettre en place un tendon, rapide, efficace et sûre.

A cet effet, le dispositif passe-tendon selon l'invention est caractérisé en ce qu'il comporte un guide d'insertion du tendon dans ledit élément de maintien.

Dans la forme de réalisation préférée de l'invention, le guide d'insertion comporte à une de ses extrémités une languette de tenue et à son autre extrémité un tronçon cylindrique creux pourvu d'une fente longitudinale et agencé pour loger une extrémité dudit tendon.

De préférence ledit guide est réalisé en une matière synthétique semi-rigide.

De façon avantageuse, l'élément de maintien dudit tendon peut comporter un filet réalisé dans un treillis cylindrique à mailles déformables de manière à passer d'une position lâche permettant une introduction aisée du tendon à une position resserrée agencée pour emprisonner ledit tendon.

Pour permettre l'insertion et l'emprisonnement du tendon, le filet est ouvert à son extrémité supérieure et fermé à son autre extrémité par une zone de section progressivement rétrécie.

Le treillis peut être réalisé en un fil de matière synthétique ou en un fil métallique.

Dans la forme de réalisation préférée, la tige souple est malléable et comporte une extrémité libre arrondie. Elle est raccordée au filet par la zone de section progressivement rétrécie et est de préférence réalisée d'une pièce avec le filet, au moyen des fils dudit filet juxtaposés soudés entre eux.

Pour faciliter la mise en place du tendon, le filet peut comporter une boucle du type lasso à son extrémité ouverte.

La présente invention et ses avantages apparaîtront mieux dans la description suivante d'un exemple de réalisation, et en référence aux dessins annexés, dans lesquels:
- la figure 1 illustre la mise en place d'un tendon dans le filet d'un dispositif passe-tendon selon l'invention,
- la figure 2 illustre une forme de réalisation du guide d'insertion du dispositif selon la figure 1,
- la figure 3 illustre le filet seul pourvu de ses moyens de guidage,
- la figure 3a illustre un détail agrandi du filet, et
- la figure 4 montre le dispositif passe-tendon selon l'invention introduit dans un modèle symbolisant un canal ossaire, les mailles du filet étant en position resserrée autour d'un tendon.

En référence aux figures, le dispositif passe-tendon représenté se compose d'une part d'un élément de maintien 20 destiné à emprisonner un tendon 10, sous la forme d'un filet 1 allongé et de forme sensiblement cylindrique, réalisé au moyen d'un treillis de mailles 2 et d'autre part d'un moyen d'insertion du tendon 10 dans le filet 1 sous la forme d'un guide 12.

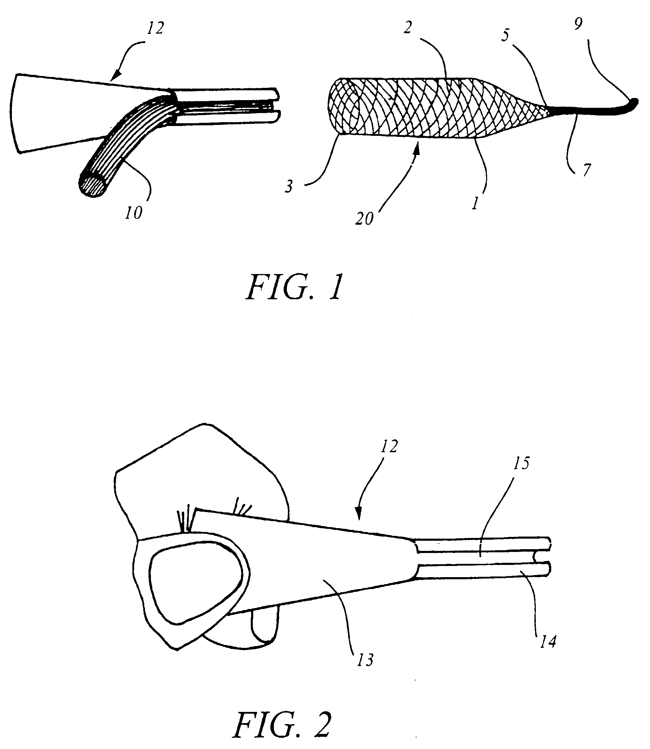

Le guide d'insertion 12, tel que représenté par la figure 2, est réalisé en une matière synthétique semi-rigide et comporte à une de ses extrémités une languette 13 permettant de le tenir et à son autre extrémité un tronçon cylindrique creux 14 agencé pour recevoir une extrémité du tendon 10. A cet effet ce tronçon 14 est pourvu sur toute sa longueur d'une fente longitudinale 15 permettant l'introduction de la pointe d'un instrument approprié, pour faire glisser le tendon 10 dans le guide 12. Ce guide a pour but de servir de "tuteur" au tendon, qui est un élément souple, lors de sa mise en place dans le filet et d'autre part d'éviter qu'il ne s'accroche dans les mailles dudit filet.

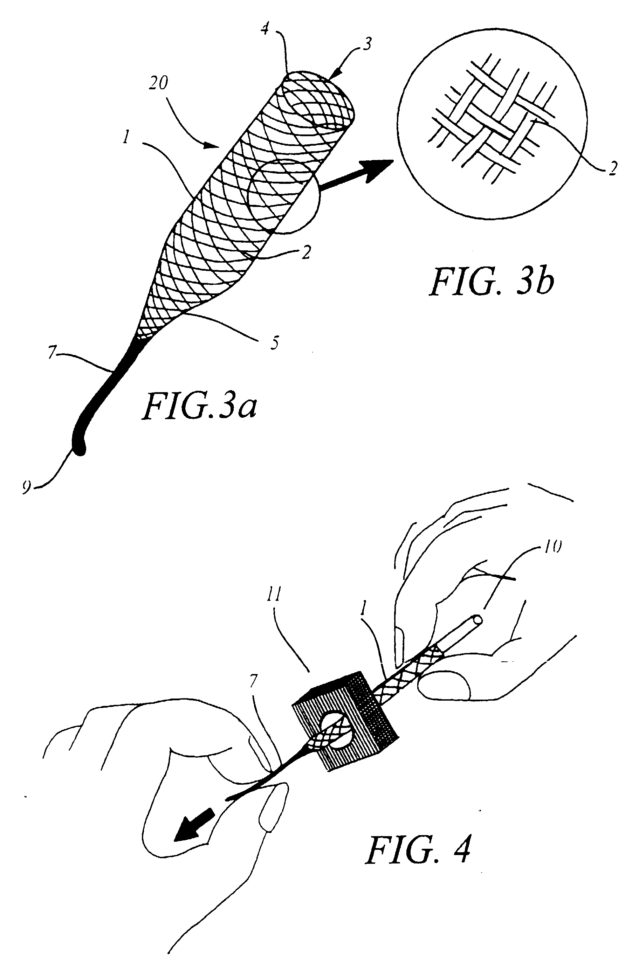

Les mailles 2 du treillis constituant le filet 1 représenté par les figures 1 et 3 sont formées de fils en matière synthétique, notamment de fils de Nylon®, de Vicryl® ou d'autres substances ayant les propriétés requises ou de fils métalliques. Comme le montre la figure 3a, les mailles de ce treillis sont lâches lorsque le filet est au repos. L'extrémité supérieure 3 du filet est ouverte et son diamètre est supérieur à celui d'un tendon lorsque le filet est au repos. L'extrémité inférieure 5 du filet est fermée et prolongée par une tige 7 qui se termine par un bout arrondi 9. Entre la tige 7, qui définit l'extrémité inférieure du filet 1 en constituant le moyen de guidage à travers le canal ossaire, et l'extrémité supérieure 3 dudit filet 1, se situe une zone de section progressivement rétrécie. L'extrémité supérieure 3 du filet contient dans ses dernières mailles une sorte de boucle de lasso 4 composée d'un fil unique, en principe du même type qui celui du filet.

La mise en place du tendon 10 dans le dispositif selon l'invention se fait de la manière suivante:
- on met le tendon en place dans le guide d'insertion 12 en l'introduisant dans le tronçon cylindrique 14 et en le faisant glisser jusqu'à l'extrémité dudit tronçon à l'aide de la pointe d'un instrument approprié passant à travers la fente 15,
- on introduit l'ensemble guide 12 + tendon 10 dans le filet 1 dont les mailles 2 sont relâchées,
- on retire le guide 12 en maintenant le tendon 10 dans le filet à l'aide de la pointe d'un instrument passée à travers les mailles du filet et de la fente 15,
- une fois le tendon introduit dans le filet, la boucle 4 est serrée autour du tendon et nouée à la main selon la manière chirurgicale habituelle. Ceci permet d'induire le resserrement du filet 1 sans que l'on ait à tenir manuellement son extrémité supérieure, ce qui est parfois difficile selon le type d'intervention en raison de l'étroitesse du champ opératoire ou de la brièveté du tendon ou de toute autre raison. La boucle 4 n'endommage pas le tendon. Au besoin, si elle gêne le passage du tendon en raison de son encombrement, en particulier de son noeud ou du renflement qu'elle provoque sur le tendon, elle peut être sectionnée et retirée juste avant son passage dans le canal ossaire, puisque à ce moment, l'extrémité du tendon a déjà passé le canal ossaire et que le reste du tendon peut donc suivre sans problèmes.

La tige 7 est réalisée dans une matière souple et malléable qui peut être déformée en gardant la forme qu'on lui donne. Cette propriété peut se révéler très utile pour passer un canal ossaire fortement coudé ou à géométrie complexe. Cette tige peut être constituée par le prolongement du filet 1, les fils du filet étant soudés les uns aux autres ou par toute autre matière synthétique appropriée. Toutefois, elle peut être indépendante des fils du filet et rapportée à ce dernier.

Lorsque le chirurgien veut remplacer un tendon rompu, dans certains cas il perce tout d'abord deux trous en V pour former le canal ossaire, comme dans la méthode classique présentée dans l'art antérieur. Il peut aussi ne percer qu'un seul trou rectiligne ou un canal arrondi à l'aide d'une pince à champ arrondie par exemple. Ensuite, grâce à sa souplesse, la tige 7 est préformée et garde la forme que le chirurgien lui a imprimée de manière à suivre la configuration du canal ossaire. Ainsi, le chirurgien peut passer très facilement la tige 7 dans ce canal.

Puis, le chirurgien "comprime" le dispositif en rapprochant l'extrémité supérieure 3 du filet du bout arrondi 9 de la tige 7. De cette manière, les mailles 2 s'ouvrent et le diamètre du filet 1 augmente. Le chirurgien peut alors introduire très aisément le tendon dans le filet 1 selon le processus déjà décrit.

Ensuite, comme le montre la figure 4, le chirurgien étire le filet en éloignant les deux extrémités 3 et 9 en tirant sur la tige 7 et en retenant l'extrémité ouverte 3 du filet 1 sur le tendon 10. De cette manière, les mailles 2 se resserrent autour du tendon 10 et le diamètre du filet 1 diminue jusqu'à ce qu'elles enserrent et emprisonnent ce tendon. Plus la traction exercée est importante, plus le tendon 10 est serré et maintenu dans le filet 1. Enfin, le chirurgien n'a plus qu'à passer l'ensemble filet-tendon 1,10 dans le canal ossaire 11. Cette opération se fait très facilement puisque l'ensemble filet-tendon est parfaitement compact. La surépaisseur due au filet disposé à la périphérie du tendon est négligeable, d'autant plus que le tendon est comprimé par le système et présente un diamètre légèrement inférieur à son diamètre habituel.

Avec le dispositif selon l'invention, l'ancrage du tendon 10 dans le filet 1 est effectué très rapidement, d'une manière très sûre et sans risque d'endommagement du tendon. Il est provisoire et ne nécessite aucun fil d'ancrage qui pourrait blesser le tendon. De plus cette manoeuvre peut être répétée plusieurs fois sans porter atteinte à l'intégrité du tendon si des passages dans plusieurs canaux ossaires ou des passages multiples dans un même canal ossaire sont nécessaires.

L'ordre des différentes manipulations effectuées pour introduire le tendon 10 dans le filet 1 peut être modifié. Il est aussi possible d'introduire par avance le guide d'insertion 12 dans le filet et ensuite d'insérer le tendon dans ledit guide.

## Revendications

1. Dispositif passe-tendon, pour passer un tendon dans un canal ossaire pouvant avoir une forme coudée, comprenant un filet (1) de maintien (20) d'une extrémité dudit tendon (10), ledit élément étant pourvu d'une tige souple (7) pour faire passer le tendon dans ledit canal ossaire (11), caractérisé en ce qu'il comporte un guide fendu d'insertion (12) dudit tendon dans ledit élément de maintien.

2. Dispositif passe-tendon selon la revendication 1, caractérisé en ce que le guide d'insertion (12) comporte à une de ses extrémités une languette de tenue (13) et à son autre extrémité un tronçon cylindrique creux (14) pourvu d'une fente longitudinale (15) et agencé pour loger une extrémité dudit tendon.

3. Dispositif passe-tendon selon la revendication 2, caractérisé en ce que le guide d'insertion (12) est réalisé en une matière synthétique semi-rigide.

4. Dispositif passe-tendon selon la revendication 1, caractérisé en ce que l'élément de maintien (20) dudit tendon est réalisé dans un treillis cylindrique à mailles déformables (2) de manière à passer d'une position lâche permettant une introduction aisée du tendon (10) à une position resserrée agencée pour emprisonner ledit tendon.

5. Dispositif passe-tendon selon la revendication 4, caractérisé en ce que ledit filet (1) est ouvert à son extrémité supérieure (3) et fermé à son autre extrémité par une zone de section progressivement rétrécie.

6. Dispositif passe-tendon selon la revendication 4, caractérisé en ce que le treillis est réalisé en un fil de matière synthétique.

7. Dispositif passe-tendon selon la revendication 4, caractérisé en ce que le treillis est réalisé en un fil métallique.

8. Dispositif passe-tendon selon la revendication 1, caractérisé en ce que la tige souple (7) est malléable et comporte une extrémité libre (9) arrondie.

9. Dispositif passe-tendon selon la revendication 5, caractérisé en ce que la tige souple (7) est raccordée audit filet (1) par la zone de section progressivement rétrécie et est réalisée d'une pièce avec ledit filet.

10. Dispositif passe-tendon selon la revendication 9, caractérisé en ce que la tige souple (7) est réalisée au moyen des fils du filet juxtaposés soudés entre eux.

11. Dispositif passe-tendon selon la revendication 5, caractérisé en ce que le filet (1) comporte une boucle (4) du type lasso à son extrémité ouverte (3).

## Claims

1. Tendon guide device used to pass a tendon through a bone canal which may be of a curved shape, comprising a net (1) constituting a holder element (20) of an end portion of said tendon (10), said element being provided with a flexible tail (7) for passing the tendon through said bone canal (11), characterized in that it comprises a split insertion guide (12) of said tendon inside said holder element.

2. Tendon guide device according to claim 1, characterized in that the insertion guide (12) comprises a tongue-like handle (13) at one end and a hollow cylindrical portion (14) at the other end with a longitudinal slit (15) designed to hold one extremity of said tendon.

3. Tendon guide device according to claim 2, characterized in that the insertion guide (12) is made of semirigid synthetic material.

4. Tendon guide device according to claim 1, characterized in that the holder element (20) of said tendon is made of a network of deformable mesh openings (2) which are adjustable between a slackened position in which the tendon (10) is easily introduced inside and a tightened position in which said tendon is tightly enclosed.

5. Tendon guide device according to claim 4, characterized in that said net (1) is open at its upper end (3) and closed at its other end by a progressively tapering portion.

6. Tendon guide device according to claim 4, characterized in that the mesh is made of filaments of synthetic material.

7. Tendon guide device according to claim 4, characterized in that the mesh is made of metal wires.

8. Tendon guide device according to claim 14 characterized in that the flexible tail (7) is a malleable tail and comprises a rounded unattached end (9).

9. Tendon guide device according to claim 5, characterized in that the flexible tail (7) is connected to said net (1) at the progressively tapering portion and forms one piece with said net.

10. Tendon guide device according to claim 9, characterized in that the flexible tail (7) is made by joining the juxtaposed filaments of the net.

11. Tendon guide device according to claim 5, characterized in that the net (1) comprises a lasso-type loop (4) at its open end (3).

## Patentansprüche

1. Sehnendurchführvorrichtung zur Durchführung einer Sehne durch einen Knochenkanal, der eine gekrümmte Form aufweisen kann, die ein Netz (1) als Halteelement (20) eines Endes der Sehne (10) aufweist, wobei das Element eine flexible Stange (7) zum Einführen der Sehen in den Knochenkanal (11) aufweist,
**dadurch gekennzeichnet,** daß
sie eine gespaltene Einführvorrichtung (12) zum Einführen der Sehne in das Halteelement aufweist.

2. Sehnendurchführvorrichtung nach Anspruch 1,
**dadurch gekennzeichnet,** daß
die gespaltene Einführvorrichtung (12) an einem ihrer Enden eine Haltelasche (13) aufweist, und an ihrem anderen Ende ein hohles zylindrisches Teil (14) aufweist, das mit einem Längsschlitz (15) versehen, und so angeordnet ist, daß ein Ende der Sehne aufnehmbar ist.

3. Sehnendurchführvorrichtung nach Anspruch 2,
**dadurch gekennzeichnet,** daß
die Einführvorrichtung (12) aus einem halbstarren, synthetischen Material besteht.

4. Sehnendurchführvorrichtung nach Anspruch 1,
**dadurch gekennzeichnet,** daß
das Halteelement (20) für die Sehne aus einem zylindrischen Gittergeflecht mit verformbaren Maschen (2) besteht, um von einer lockeren Position, die eine leichtere Einführung der Sehne (10) ermöglicht, in eine stärker angespannte Position zu gelangen, um die Sehne festzuhalten.

5. Sehnendurchführvorrichtung nach Anspruch 4,
**dadurch gekennzeichnet,** daß
das Netz (1) an seinem oberen Ende (3) offen, und an seinem anderen Ende durch eine im Querschnitt fortlaufend verengte Zone geschlossen ist.

6. Sehnendurchführvorrichtung nach Anspruch 4,
**dadurch gekennzeichnet,** daß
das Gittergeflecht in Form eines Netzes aus synthetischem Material ausgeführt ist.

7. Sehnendurchführvorrichtung nach Anspruch 4,
**dadurch gekennzeichnet,** daß
das Gittergeflecht in Form eines metallischen Netzes ausgeführt ist.

8. Sehnendurchführvorrichtung nach Anspruch 1,
**dadurch gekennzeichnet,** daß
die flexible Stange (7) geschmeidig ist, und ein freies, abgerundetes Ende (9) aufweist.

9. Sehnendurchführvorrichtung nach Anspruch 5,
**dadurch gekennzeichnet,** daß
die flexible Stange (7) durch eine im Querschnitt fortlaufend verengte Zone mit dem Netz (1) verbunden ist, und einstückig mit dem Netz ausgeführt ist.

10. Sehnendurchführvorrichtung nach Anspruch 9,
**dadurch gekennzeichnet,** daß
die flexible Stange (7) mit Hilfe von nebeneinander angeordneten Fäden des Netzes, die miteinander verschweißt sind, ausgeführt ist.

11. Sehnendurchführvorrichtung nach Anspruch 5,
**dadurch gekennzeichnet,** daß
das Netz (1) an seinem offenen Ende (3) eine lassoartige Schleife (4) aufweist.
